## Europäisches Patentamt

## European Patent Office

## Office européen des brevets

(19)

(11) Publication number: **0 047 647**
**B1**

(12) **EUROPEAN PATENT SPECIFICATION**

(45) Date of publication of patent specification: **27.12.85**

(21) Application number: **81304053.2**

(22) Date of filing: **04.09.81**

(51) Int. Cl.⁴: **A 61 K 9/06,** A 61 K 9/08,
A 61 K 31/19, A 61 K 31/195

(54) **Pharmaceutical compositions for promoting the healing of wounds.**

(30) Priority: **04.09.80 GB 8028554**

(43) Date of publication of application:
**17.03.82 Bulletin 82/11**

(45) Publication of the grant of the patent:
**27.12.85 Bulletin 85/52**

(84) Designated Contracting States: .
**DE FR GB NL SE**

(56) References cited:
DE-A-2 438 703
GB-A-1 397 680
US-A-3 786 076

CHEMICAL ABSTRACTS, volume 93, nr. 20,
November 17, 1980, ref. 191909f, page 351
COLUMBUS, OHIO (US)
HAGERS HANDBUCH DER
PHARMAZEUTISCHEN PRAXIS, 4th Edition
Springer Verlag, 1971, volume VII BERLIN (DE)
"Arzneiformen und Hilfsstoffe, T1. A.
Arzneiformen"

(73) Proprietor: **Ed. Geistlich Söhne A.G. für**
**Chemische Industrie**
**CH-6110 Wolhusen Lucerne (CH)**

(72) Inventor: **Cottier, David**
**Langdale 10 Plemstall Way**
**Mickle Trafford, Chester CH2 4Q5 (GB)**
Inventor: **Stanley, John Knowles** .
**Rydal Mount, Ruff Lane**
**Ormskirk, Lancashire (GB)**
Inventor: **Calver, Richard Frederick**
**West Acre, 27 Howbeck Road**
**Oxton, Birkenhead L43 6UL (GB)**

(74) Representative: **Cockbain, Julian et al**
**Frank B. Dehn & Co. Imperial House 15-19,**
**Kingsway**
**London WC2B 6UZ (GB)**

(56) References cited:
CHEMICAL ABSTRACTS, volume 82, no. 16,
April 21, 1975, ref. 103149c, page 358
COLUMBUS, OHIO (US)

Courier Press, Leamington Spa, England.

## Description

The present invention relates to pharmaceutical compositions for promoting the healing of wounds.

Patients with multiple injuries, especially those with large third degree burns and those undergoing major surgical operations, frequently need intravenous feeding. The nutrients can reach the reparative area if that area is well vascularised, but if adequate vascularisation is lacking, the only way for nutrients to reach the site under repair is by diffusion from neighbouring vascularised regions. If the areas to be repaired are large, the regenerative capacity of the patient may be exceeded, resulting in poorly granulating, easily infected surfaces. When large infected wounds do not respond to intensive nursing and medical care, the distress to both patient and nursing attendants is very great.

It has been found that wound healing can be promoted by application of nutrients directly to the site concerned. The theoretical basis for such uptake of nutrients directly by the growing cells is not, however, well understood. Previous proposals of this kind have required application of a solution of nutrients, principally amino acids and carbohydrates, by infusion onto the surface, preferably using a dressing to maintain a body of liquid in contact with the wound.

We have now found that a particular type of nutrient solution in the form of an aqueous semi-solid gel is especially suitable for the promotion of wound healing by direct alimentation. By the term "semi-solid gel" we mean a gel which is spreadable or smearable, having the consistency of an ointment or cream. This term is intended to include colloidal dispersions of such materials as carboxymethyl cellulose, agarose, which have little or no resilience or elasticity but are of a gel-like nature. Compositions of such a consistency are particularly beneficial in releasing nutrients into the wound, as compared with solid or rigidly gelatinous nutrient formulations.

We have found that pH of such a composition is advantageously neutral or slightly alkaline, i.e. in the range 6.5 to 8.0. This is in contrast to a number of intravenous nutrient solutions which have generally previously been acidic.

According to the present invention we provide a semi-solid aqueous gel composition for wound alimentation containing in solution L-isoleucine, L-leucine, L-lysine, L-methionine, L-phenylalanine, L-threonine, L-tryptophan, L-valine, L-arginine, L-histidine, L-alanine and malic acid, the pH of the composition being in the range 6.5 to 8, characterized in that said composition is in semi-solid aqueous gel form.

According to a further aspect of the present invention we provide a method of manufacturing a semi-solid aqueous gel composition for wound alimentation which method comprises mixing L-isoleucine, L-leucine, L-lysine, L-methionine, L-phenylalanine, L-threonine, L-tryptophan, L-valine, L-arginine, L-histidine and L-alanine together with malic acid and adjusting the pH of the composition to within the range 6.5 to 8.

In general, the total weight of amino acids should be in the range 3—17%. However, the overall quantity of amino acid material partly depends on the nature of the individual amino acids. In many intravenous nutrient solutions, the total nitrogen supplied is predominantly in the form of simple amino acids such as glycine, alanine and glutamic acid. The body is capable of using these amino acids to synthesise a number of the non-essential amino acids present in human tissue. In general, however, this approach is less efficient than using a mixture of amino acids which approximates relatively closely to the proportions of the amino acids found in human tissues. It seems likely that the mechanisms available to the body for synthesis of other amino acids from glycine, alanine and glutamic acid may not be effective at the site of granulation of new tissue. For that reason, the amino acids are, in one embodiment of the invention, preferably in the proportions found in human tissue and in that it is not intended that the quantities of glycine, alanine and glutamic acid should be high enough to provide significant levels of other amino acids, the overall concentration of amino acids in the gel according to the invention is preferably in the range 3 to 6% by weight.

It should be noted, however, that although the primary task of wound healing may be to regenerate skin, which consists largely of fibrous tissue, crude amino acid preparations derived by hydrolysis of collagen or elastin, which may be relatively inexpensive, do not contain all the essential amino acids and may contain peptide materials giving rise to reactions at the site of application. Thus it is preferred that the compositions of the present invention are free from proteins or peptides.

When the gel is hypertonic, due to relatively high concentrations of amino acids, the wound healing process may be promoted due to increased absorption of liquid secretion into the gel and increased movement of amino acids out of the gel. On the other hand, where the gel is substantially isotonic, it is more compatible with the granulating tissue. The amino acids used in the gel formulations of the invention are preferably present in the following quantities in g/kg gel.

| | | |
|---|---|---|
| L-Isoleucine | 1.3—5.7 | |
| L-Leucine | 2.1—8.8 | |
| L-Lysine HCl | 3.1—8.5 | |
| L-Methionine | 1.8—7.5 | The Essential amino acids |
| L-Phenylalanine | 3.2—7.5 | |
| L-Threonine | 1.0—4.0 | |
| L-Tryptophan | 0.5—1.7 | |
| L-Valine | 1.6—6.7 | |
| L-Arginine | 2.1—9.7 | The Semi-essential amino acids |
| L-Histidine | 1.1—5.8 | |
| L-Alanine | 2.9—15.6 | |
| L-Glutamic acid | 1.9—21.6 | |
| Glycine | 2.0—25.0 | |
| L-Proline | 2.0—15.0 | |
| L-Ornithine-L-Aspartate | 1.0—8.5 | |
| L-Serine | 1.6—7.3 | |

As indicated above, all the essential and semi-essential amino acids are present in the gel.

A preferred range of amino acid concentrations in g/kg gel is as follows:—

| | |
|---|---|
| L-Isoleucine | 1.8—5.4 |
| L-Leucine | 2.7—8.3 |
| L-Lysine HCl | 3.2—9.6 |
| L-Methionine | 2.2—6.8 |
| L-Phenylalanine | 3.2—9.8 |
| L-Threonine | 1.5—4.5 |
| L-Tryptophan | 0.6—2.0 |
| L-Valine | 2.1—6.3 |
| L-Arginine | 4.3—12.9 |
| L-Histidine | 1.0—3.2 |
| L-Alanine | 4.7—14.1 |
| L-Glutamic acid | 0.9—2.9 |
| Glycine | 2.0—6.2 |
| L-Proline | 5.6—16.9 |
| L-Ornithine-L-Aspartate | 0.9—2.9 |
| L-Serine | 1.1—3.5 |

The nutrient compositions of the invention should also contain essential trace of minerals, in particular sodium, potassium, magnesium, chloride and acetate ions.

A particularly important component in the nutrient gel according to the invention is malic acid. This is directly beneficial in the regenerative process. The concentration of malic acid in the medium is preferably in the range 0,2—0,6% by weight. The pH of the medium is preferably approximately neutral, i.e. in the range 6.5—8, preferably 7.0—7.5. It will be appreciated that in this pH range, many of the amino acids and the malic acid will be present partially in the form of salts, e.g. alkali metal salts such as sodium salts.

The gel-forming agent may be of a type conventional in the pharmaceutical art. Sodium carboxymethylcellulose is particularly suitable. Other cellulose derivatives such as microcrystalline cellulose are also suitable, as well as polysaccharides such as alginate, agarose, tragacanth, and polyvinylpyrrolidone.

A surprising finding is that the compositions of the invention are resistant to bacterial and fungal infection in spite of the relatively high concentrations of nutrient material present. This is contrary to previous experience in handling nutrient materials. In some cases actual antibacterial activity has been observed. Thus, our tests have shown the compositions according to the invention to inhibit growth of *Candida albicans, Clostridium sporogenes, Pseudomonas aerogenes* and *Escherischia coli* and to be bactericidal with respect to *Staphylococcus aureus*.

The new compositions are intended for application to the surface to be treated at relatively long intervals, for example, every 12 hours. Such a regime is similar to that proposed when using liquid solutions of amino acids by a continuous infusion technique and it is surpising that the gel can contain sufficient amino acid material to sustain increased granulation over such a long period. It will be appreciated that it is a significant advance in such treatment that it is possible to dispense with the container for liquid nutrient and its necessary support system and tubing as previously used with the proposed infusion technique.

In view of the significant danger of infection of large wound areas, it is often desirable to interpose a wound-disinfection treatment between applications of the gel material according to the invention. Particularly suitable antibacterial substances for this purpose are Noxythioline, Taurolidine, iodine-providone, chlorhexidine and antibiotics suitable for local administration such as amfomycin, bacitracin, neomycin, tyrothricin, ploymycin, as well as tetracyclines and amino glycosides.

The following example is given by way of illustration.

Example
1.0 kg of gel contains:

| | g |
| --- | --- |
| Sodium-carboxy-methyl-cellulose | 13,000 |
| Glycerol BP | 10,000 |
| Propylenglycol | 10,000 |
| L-Isoleucine | 3,586 |
| L-Leucine | 5,444 |
| L-Lysine HCl | 6,382* |
| L-Methionine | 4,506 |
| L-Phenylalanine | 6,459 |
| L-Threonine | 2,998 |
| L-Tryptophan | 1,315 |
| L-Valine | 4,206 |
| L-Arginine | 8,635 |
| L-Histidine | 2,060 |
| L-Alanine | 9,380 |
| L-Glutamic acid | 1,876 |
| Glycine | 4,129 |
| L-Proline | 11,265 |
| L-Ornithine-L-Aspartate | 1,876 |
| L-Serine | 2,253 |
| | 109,370 |

| | mmol |
| --- | --- |
| Sodium ions $Na^+$ | 32,87 |
| Potassium ions $K^+$ | 28,04 |
| Magnesium ions $Mg^{++}$ | 2,34 |
| Chloride ions $Cl^-$ | 63,04 |
| Acetate ions $Ac^-$ | 4,68 |
| Malic acid | 4,318 |
| Aqua dest. ad | 1000,000 |

*(Base 5,016)

The glycerol and propylene glycol are added to the sodium carboxymethylcellulose and mixed to produce a smooth dispersion. The amino acids, trace minerals and malic acid are dissolved in most of the water and the pH is adjusted to 7.2 with sodium hydroxide. The above dispersion is added to the amino acid solution with continuous stirring, until free from undispersed solids and entrained air. The remaining water is then added to make up to the final volume. The resulting transparent, semi-solid gel is then autoclaved at 115°—118°C for at least 30 minutes. The consistency at 20°C is about 20,000 mPa·s (by Brookfield Viscometer).

**Claims**

1. A composition for wound alimentation containing in solution L-isoleucine, L-leucine, L-lysine, L-methionine, L-phenylalanine, L-threonine, L-tryptophan, L-valine, L-arginine, L-histidine, L-alanine and malic acid, the pH of the composition being in the range 6.5 to 8, characterized in that said composition is in semi-solid aqueous gel form.

2. A composition as claimed in claim 1 further containing the amino acids L-glutamic acid, glycine, L-proline, L-serine, L-ornithine and L-aspartic acid.

3. A composition as claimed in claim 1 or claim 2 in which the gel is free from proteins or peptides.

4. A composition as claimed in claim 3 containing as a gel-forming agent, carboxymethyl cellulose, microcrystalline cellulose, alginate, agarose, tragacanth or polyvinylpyrrolidone.

5. A composition as claimed in any of the preceding claims having a pH in the range 7.0—7.5.

6. A composition as claimed in any of the preceding claims in which the concentration of amino acids is 3 to 6% by weight.

7. A composition as claimed in any of the preceding claims in which the concentration of malic acid is 0.2 to 0.6% by weight.

8. A method of manufacturing a semi-solid aqueous gel composition for wound alimentation which method comprises mixing L-isoleucine, L-leucine, L-lysine, L-methionine, L-phenylalanine, L-threonine, L-tryptophan, L-valine, L-arginine, L-histidine and L-alanine together with malic acid and adjusting the pH of the composition to within the range 6.5 to 8.

**Revendications**

1. Composition accélérant la guérison de blessures contenant en solution de la L-isoleucine, de la L-leucine, de la L-lysine, de la L-méthionine, de la L-phényl-alanine, de la L-thréonine, du L-tryptophane, de la L-valine, de la L-arginine, de la L-histidine, de la L'alanine et de l'acide malique, le pH de la composition étant compris entre 6,5 et 8, caractérisée en ce que la composition est sous lu forme d'un gel aqueux semi-solide.

2. Composition suivant la revendication 1, comprenant, en outre, les aminoacides que sont l'acide L-glutamique, la glycine, la L-proline, la L-sérine, la L-ornithine et l'acide L-aspartique.

3. Composition suivant la revendication 1 ou la revendication 2, dans laquelle le gel est exempt de protéines ou de peptides.

4. Composition suivant la revendication 3, contenant comme agent gélifiant de la carboxy-méthylcellulose, de la cellulose microcristalline, un alginate, de l'agarose, de la gomme adragante ou de la polyvinylpyrrolidone.

5. Composition suivant l'une quelconque des revendications précédentes ayant un pH compris entre 7,0 et 7,5.

6. Composition suivant l'une quelconque des revendications précédentes, dans laquelle la concentration d'aminoacides est comprise entre 3 et 6% en poids.

7. Composition suivant l'une quelconque des revendications précédentes, dans laquelle la concentration d'acide malique est comprise entre 0,2 et 0,6% en poids.

8. Procédé de fabrication d'une composition de gel aqueux semi-solide pour accélérer la guérison des blessures, ce procédé consistant à mélanger de la L'isoleucine, de la L-leucine, de la L-lysine, de la L-méthionine, de la L-phényl-alanine, de la L'thréonine, du L-tryptophane, de la L-valine, de la L-arginine, de la L-histidine et de la L-alanine à de l'acide malique et à ajuster le pH de la composition entre 6,5 et 8.

**Patentansprüche**

1. Mittel zur Wundversorgung, das in Lösung L-Isoleucin, L-Leucin, L-Lysin, L-Methionin, L-Phenylalanin, L-Threonin, L-Tryptophan, L-Valin, L-Arginin, L-Histidin, L-Alanin und Apfelsäure enthält, wobei der pH des Mittels zwischen 6,5 und 8 liegt, dadurch gekennzeichnet, daß das Mittel ein semi-festes wäßriges Gel ist.

2. Mittel nach Anspruch 1, das außerdem die Aminosäuren L-Glutaminsäure, Glycin, L-Prolin, L-Serin, L-Ornithin und L-Asparaginsäure enthält.

3. Mittel nach Anspruch 1 oder 2, bei dem das Gel keine Proteine oder Peptide enthält.

4. Mittel nach Anspruch 3, das als gel-bildendes Agens Carboxymethylcellulose, microkristalline Cellulose, Alginat, Agarose, Traganth oder Polyvinylpyrrolidon enthält.

5. Mittel nach einem der vorhergehenden Ansprüche, mit einem pH von 7,0 bis 7,5.

6. Mittel nach einem der vorhergehenden Ansprüche, bei dem die Aminosäurenkonzentration 3—6 Gew.-% beträgt.

7. Mittel nach einem der vorhergehenden Ansprüche, bei dem die Konzentration an Äpfelsäure 0,2 bis 0,6 Gew.-% beträgt.

8. Verfahren zur Herstellung eines Mittels zur Wundversorgung in Form eines semi-festen wäßrigen Gels, wobei man L-Isoleucin, L-Leucin, L-Lysin, L-Methionin, L-Phenylalanin, L-Threonin, L-Tryptophan, L-Valin, L-Arginin, L-Histidin, und L-Alanin mit Äpfelsäure mischt und den pH des Mittels auf 6,5 bis 8 einstellt.